# EUROPEAN PATENT SPECIFICATION

(11) **EP 4 509 032 B1**
(45) Date of publication and mention of the grant of the patent: **11.11.2026**
(21) Application number: 23192177.6
(22) Date of filing: 18.08.2023
(51) Int. Cl.: A61B 1/005, A61B 1/012, A61M 25/01, A61B 1/008, A61B 1/018

(54) **ENDOSCOPE WITH OFF-CENTRE HINGES FOR BENDING SECTION**
ENDOSKOP MIT NICHTZENTRISCHEN SCHARNIEREN FÜR BIEGEABSCHNITT
ENDOSCOPE À CHARNIÈRES EXCENTRÉES POUR SECTION DE PLIAGE

(43) Date of publication of application: 19.02.2025
(73) Proprietor: Ambu A/S, 2750 Ballerup (DK)
(72) Inventor: BUCH, Ken Henrik, 4760 Vordingborg (DK); HANSEN, Frederik Clausager Vemb, 2400 Copenhagen NV (DK)
(74) Representative: Winter, Brandl - Partnerschaft mbB

(56) References cited:
- WO-A1-2022/106403
- US-A1- 2013 116 705
- US-A1- 2019 328 212
- US-A1- 2021 393 113

## Description

The present disclosure relates to an endoscope comprising a proximal endoscope handle or interface, an insertion cord extending along an endoscope axis from the endoscope handle or interface and configured to be inserted into a patient's body cavity, the insertion cord comprising an insertion tube, a bending section and a distal tip unit, and a steering mechanism configured to swivel the distal tip unit by bending the bending section at least in a first bending plane; wherein the bending section comprises a number of segments aligned along the endoscope axis and forming a cable lumen portion for accommodation of at least one electrical cable.

### Related Art

Steerable endoscopes often have a proximal endoscope handle and a distal insertion cord including a bending section and a distal tip unit. In order to deflect the distal tip unit, the bending section can be bent or manipulated by pulling one or more pulling/ steering wires, which extend into the insertion cord of the endoscope, and which have distal portions attached to the bending section. The bending section usually has segments, which are connected via hinges and form a lumen or groove for at least one cable, such as a camera cable.

Typically, in an endoscope, which allows two-way-bending, two pulling wires may be provided, which are circumferentially offset with respect to each other by 180°, and pulling either one of the pulling wires/ steering wires will result in a bending motion in a corresponding direction. Thus, one degree of freedom (one bending plane) is provided comprising an up/ down movement or a left/ right movement of the bending section effected by pulling one of two diametrically opposed pulling wires. Proximally, the two pulling wires are often connected to one control wheel to operate the pulling wires.

Similarly, endoscopes, which allow four-way-bending generally comprise four pulling wires circumferentially offset by 90°. In this case, one pair of diametrically opposed pulling wires operates the up/ down movement of the bending section (i.e. in a first bending plane/ providing a first degree of freedom) e.g. via one control wheel. The other two diametrically opposed pulling wires may operate the left/ right movement of the bending section (i.e. in a second bending plane/ providing a second degree of freedom), e.g. via another control wheel.

Insertion cords of endoscopes usually have diameters, which are as small as possible and/ or which provide room for large working channels so that relatively large instruments may be inserted through said working channels. In particular for endoscope insertion cords with a small outer diameter, there is not enough space in the bending section to have hinges and electrical cables (in short: cable) in the same plane. If the cable is displaced from a centre plane, the bending of the bending section will result in an interchanging pull and push on the cable, said pull and push resulting in bulges and potential damage to the cable.

One example for such an endoscope can be found in EP 2 941 174 B1. Said endoscope comprises an articulated tip part that may be moulded as a one-piece polypropylene member and has a number of segments interconnected by means of flexible hinge members extending in one plane. The segments have a generally circular cross-section with several separate passages, with one of said passages being adapted to accommodate the electrical supply wires for the camera and the light emitting diodes, signal wires for the camera, etc.

Further, EP 3 925 513 A1 discloses an endoscope with an articulated bending section body comprising a number of segments forming a central passage and being connected by hinge parts aligned in one plane. A cross-sectional shape of the passage comprises a number of sectors for a working channel, electrical cables, light fibres etc.

Various other designs for flexible sections of endoscope insertion cords are known from the prior art. For example, US 6 749 560 B1 and US 6 780 151 B2 respectively disclose an endoscope comprising a control section and a shaft extending from the control section. The shaft has a tubular frame with slots, which extend into the tubular frame from opposite directions perpendicular to a centre axis of the tubular frame, the slots on opposed sides of the tube being offset from one another along an axial direction.

US 2022 0 338 718 A1 relates to a patterned tube for use with a scope including at least one flexible section. The at least one flexible section can include a plurality of first cuts having a first thickness, and a plurality of second cuts having a second thickness. Particular ones of the plurality of second cuts can be located substantially opposite particular ones of the plurality of first cuts and form forked ends extending on either side of the opposite particular ones of the plurality of first cuts. The at least one flexible section can further include a plurality of bending moment transfer portions. Particular ones of the bending moment transfer portions can be located between particular ones of the first cuts and particular ones of the second cuts.

Moreover, US 2023 0 032 338 A1 discloses a steerable instrument for endoscopic and/or invasive type of applications, such as in surgery, which has an elongated tubular body having several flexible zones. In one embodiment, a part of a cylindrical tube to become flexible has been provided with a number of short slits divided into groups, the slits in each group being located in the same line extending perpendicular to the axis of the cylindrical element. The slits of two neighbouring groups are offset.

From US 8 007 434 B2, a shaft for an endoscope is known, portions of which have a geometry of openings through a shaft wall, said geometry contributing to a flexibility of the shaft. Said geometry is created by a pattern of shaft wall sections forming vertical bars and horizontal bars defining openings through the shaft wall.

Further, WO 2016/ 172 706 A1 and WO 2017/ 083 257 A1 disclose a steerable medical device with a tubular member including a steerable portion, which includes substantially straight slots that are substantially perpendicular to a longitudinal axis of the tubular member, but each is offset relative to the adjacent slot so that a resulting spine has a sinuous shape extending along the length of the steerable portion.

Additionally, US 2023/0165446 A1 discloses a medical device including a handle, a shaft extending from the handle, four articulation wires coupled to the handle, and an articulation member at a distal end of the shaft. First and second articulation wires lie within a first bending plane, and third and fourth articulation wires lie within a second bending plane. The articulation member includes a plurality of links arranged about the central longitudinal axis, a first link of the plurality of links is attached to an adjacent, second link of the plurality of links via a first pair of hinges, and a first hinge of the first pair of hinges is arranged on a first side of the first bending plane, and a second hinge of the first pair of hinges is located on a second side of the first bending plane, opposite the first side.

US 2019/328212 A1 discloses a bending portion for an endoscope, configured with a plurality of bending pieces, with two protruding portions in 180° offset to each other, each configured to contact an adjacent segment. The protruding portions of adjacent bending pieces are arranged in circumferential offset of preferably 90°. Additionally, apart from through holes for wires, space for long items extending in the longitudinal axis (e.g. image pick-up cables, wires, light guides) is provided within the bending pieces.

WO 2022/106403 A1 discloses an endoscope with, inter alias, a bending section comprising a plurality of segments. Adjacent segments are connected to each other by flexible hinge members, allowing bending in two bending planes perpendicular to each other. Additionally, the bending section comprises an inner lumen and a steering wire lumen. The inner lumen is disclosed to be configured for accommodating the working channel.

In all of these documents, electrical cables will be displaced from a centre plane in order to leave sufficient space for hinges and the like. Due to this, there will be an interchanging pull and push on the electrical cable when the bending section is bent back and forth, as described above.

### Brief description of the disclosure

**In** view of the above-described problems it is an object of the present disclosure to provide an endoscope, which shall reduce or avoid the disadvantages of the related art. **In** particular, it is an object of the disclosure to provide a steerable endoscope with an insertion cord having a bending section, which allows the provision of a particularly large working channel and reduces strain on electrical cables.

This object is solved by an endoscope in accordance with claim 1 and by a system in accordance with claim 13. Advantageous aspects of the present disclosure are claimed in the dependent claims and/or are explained below.

**In** detail, the present disclosure relates to an endoscope comprising: a proximal endoscope handle or interface; an insertion cord extending along an endoscope axis from the endoscope handle or interface and configured to be inserted into a patient's body cavity, the insertion cord comprising an insertion tube, a bending section and a distal tip unit; and a steering mechanism configured to swivel the distal tip unit by bending the bending section at least in a first bending plane; wherein the bending section comprises a number of segments aligned along the endoscope axis and forming a cable lumen portion for accommodation of at least one electrical cable, said cable lumen portion being offset from the endoscope axis and arranged in a cable plane, which is essentially perpendicular to the first bending plane, and the segments are connected by at least one first hinge device and at least one second hinge device, which define the first bending plane and are configured to allow bending of the bending section in the first bending plane, with at least one of the segments being connected to a respectively adjacent one of the segments by a first hinge device defining a first hinge axis, which is offset from the cable plane in a first direction, and at least another one of the segments being connected to a respectively adjacent one of the segments by a second hinge device defining a second hinge axis, which is offset from the cable plane in a second direction opposite to the first direction.

Expressed in other words, an endoscope with an insertion cord having a distal tip unit and a bending section for swivelling the distal tip unit in a bending section is provided, wherein the bending section has a number of segments that are hinged to each other. Corresponding hinge devices comprise at least one first hinge device and at least one second hinge device, which are offset from each other with a cable plane arranged between them. When the bending section is bent in the first bending plane, the segments are tilted relative to each other via the at least one first and second hinge devices (i.e. the at least one first hinge device and the at least one second hinge device). The first hinge device connects at least one pair of the segments and the second hinge device connects another pair of the segments, i.e. not the same pair of segments as the first hinge device. That is, two adjacent ones of the segments are connected either by the first hinge device or by the second hinge device and not by the first and the second hinge device.

In this case, the pairs of the segments may be two separate or overlapping pairs of the segments. I.e. in separate pairs of the segments, one pair may include two segments that are different from two other segments forming another pair. E.g., one pair may be formed by first and second ones of the segments and another pair may be formed by third and fourth ones of the segments. In this case, the two pairs of segments may be connected via e.g. a third or fourth hinge device for four-way bending, which will be explained later in the description. In overlapping pairs, an intermediate one of the segments may form a pair with a distally adjacent one of the segments and may further form another pair with a proximally adjacent one of the segments. E.g., one pair may be formed by first and second ones of the segments and another pair may be formed by second and third ones of the segments.

One could also say that adjacent ones of the segments are respectively connected via hinge devices (first and second hinge devices), with some of the hinge devices being arranged selectively on one side of the cable plane, i.e. to one side of the cable lumen portion, and some of the hinge devices being arranged on another side of the cable plane/ the cable lumen portion. Particularly preferably, the first and second hinge devices are arranged alternatingly along the endoscope axis, i.e. the segments are alternatingly connected by first hinge devices and second hinge devices. That is, two of the segments are connected via one of the first hinge devices and the next two segments are connected via one of the second hinge devices, and so forth. This allows for a particularly balanced bending of the bending section and reduces irregularities (e.g. a corrugated or wave-like plane) in the bending plane and the neutral plane during bending of the bending section. Thus, bulging and potential damage of the electrical cable is further reduced. Optionally, some of the segments may also be connected by other structures such as third hinge devices discussed further below. That is, the hinge devices may essentially form a spine meandering from one side of the cable plane to another side of the cable plane via the hinge devices (i.e. first, second and optionally third hinge devices).

Expressed in still other words, the segments may be separated by gaps or slits, e.g. formed by cutting, with at least a first one of the gaps or slits being bridged by a first hinge device and at least a second one of the gaps or slits, which is axially offset/ displaced from the first one of the gaps or slits, being bridged by a second hinge device. The first hinge device and the second hinge device may be circumferentially offset from each other on opposing sides of a cable plane. Preferably, the gaps or slits extend in a plane essentially orthogonal to the endoscope axis. Optionally, at least one web-like wall structure may be left between adjacent ones of the segments to form the first hinge device or the second hinge device.

The cable plane may be a plane, which coincides with or extends near a first neutral plane, which is essentially neutral with regard to a bending of the bending section in the first bending plane. "Near" in this case means that the cable plane and the first bending plane are offset from each other by a maximum offset of 50%, preferably 30%, further preferably 10%, of a diameter of the bending section. Most preferably, the cable plane is the same as the first neutral plane. The first neutral plane is determined as follows: When the bending section is bent in the first bending plane, a length of the bending section on an inner curvature side is shortened. Further, a length of the bending section on an outer curvature side is lengthened. Intermediately, perpendicular to the first bending plane and preferable in a centre of the bending section, there is a plane in which a length of the bending section remains the same in the neutral state and in a bent state of the bending section. Said plane is called the neutral or central plane. The neutral plane may be defined by the first and second hinge devices, in particular arranged between the first and second hinge devices, preferably centrally between them. In particular, the first hinge axis may be defined by parameters such as a position of the first hinge axis at the bending section and by a bending stiffness, e.g. defined by a shape and thickness of material, of the first hinge device. The second hinge axis may be similarly defined by the second hinge device. Depending on said parameters, the first and/ or second hinge axis may be inclined or parallel with respect to each other and to the neutral plane or cable plane. E.g., the first and second hinge axis may extend in a V-shape with respect to each other. Together, the first and second hinge axis of the at least one first and second hinge devices may define the neutral plane or cable plane.

The endoscope is preferably a single-use endoscope. In the insertion cord, the insertion tube, the bending section and the distal tip unit are particularly arranged in this order starting from the proximal endoscope handle or interface. The endoscope axis is a central axis of the insertion cord, with the insertion cord extending along said endoscope axis. Expressions such as "axially", "radially" and "circumferentially" relate to directions in relation to said endoscope axis. The bending plane is a plane extending along the endoscope axis and staying constant during a bending action. I.e. when the bending section is bent in the first bending plane, it moves within the bending plane and the distal tip unit is swivelled in the bending plane/ moves within the bending plane, without the bending plane itself being bent. In other words, the distal tip section is swivelled in a first or an opposing second direction by bending the bending section in the first bending plane, with the first and the second direction coinciding with/ extending in the first bending plane.

Two elements being offset from each other means that they are displaced from one another by an angular and/ or linear displacement or distance, said angular and/ or linear displacement or distance being larger than zero. The first hinge axis and the second hinge axis may be offset from the cable plane by a predetermined distance or angle. A direction of the offset of the first hinge axis with respect to the cable plane is opposite to a direction of the offset of the second hinge axis. The first and second hinge devices (i.e. the first hinge device and the second hinge device) are preferably arranged symmetrically with respect to the cable plane. The first and second hinge devices allow tilting of the correspondingly adjacent segments with respect to each other around the first hinge axis or respectively the second hinge axis. In particular, the first and second hinge devices are provided only for bending the bending section in the first bending plane, not in additional bending planes. A bending of the bending section in other bending planes may be essentially independent from the first and second hinge devices and may be provided via additional hinge devices, as discussed in more detail further below.

In particular, the endoscope comprises the electrical cable extending through the segments of the bending section. The electrical cable is further preferably a cable supplying electrical power and/ or control signals to the distal tip unit, particularly to an optical unit/ camera module arranged at the distal tip unit.

The optical unit may be a camera chip and/ or illumination means. I.e. the electrical cable may comprise one or more wires for transfer of signals such as sensory data and/ or control commands and/ or electrical energy.

The steering mechanism may include at least one steering wire, preferably two steering wires for bending of the bending section in the first bending plane. A distal end of the at least one steering wire is preferably connected at a distal end portion of the endoscope, particularly a distal end of the bending section. Further, the steering mechanism preferably comprises a control wheel arranged in or at the endoscope handle and being connected to a proximal end of the at least one steering wire to drive the bending of the bending section in the first bending plane via the at least one steering wire.

Advantageously, the endoscope as described above will allow placing of the electrical cable in or near a plane that is essentially neutral with respect to a bending action of the bending section in the first bending plane. Thus, a lengthening or compressing of the electrical cable due to the bending will be reduced, preferably minimized. In detail, only very small and local lengthening and compressing between the first and second hinge devices due to the alternatingly offset position of said first and second hinge devices will be applied to the electrical cable. Accordingly, a risk for damaging the electrical cable will advantageously be reduced.

Preferably, the first hinge device, particularly the first hinge axis, is offset from the cable plane by less than half a diameter of the bending section, preferably by a maximum of 40 %, further preferably by a maximum of 30 %, further preferably by a maximum of 20% of the diameter of the bending section. Further preferably, the second hinge device, particularly the second hinge axis, is offset from the cable plane by less than half a diameter of the bending section, preferably by a maximum of 40 %, further preferably by a maximum of 30 %, further preferably by a maximum of 20% of the diameter of the bending section. The offset mentioned above is a maximum distance of the first or respectively second hinge device, particularly the first or second hinge axis, with respect to the cable plane in a direction perpendicular to the cable plane. Alternatively, one can say that, preferably, each of the at least one first hinge device, particularly the first hinge axis, and the at least one second hinge device, particularly the second hinge axis, is at an angular position offset from the cable plane by less than 90°, preferably by a maximum of 60°, further preferably a maximum of 45°, further preferably a maximum of 30°, further preferably a maximum of 20°.

In particular, it is preferred that the first and/ or second hinge devices are formed directly adjacent to the cable lumen portion, preferably such that the first and/ or second hinge devices form part of a cable lumen wall. Thus, irregularities in an extension of the neutral plane or cable plane can be minimized particularly well. Additionally and synergistically, a good support of the electrical cable in the cable lumen portion may be achieved even in an area between the segments. This is particularly easy to achieve, when the first and second hinge devices form webs or bridges of material between the segments.

Further preferably, the first hinge device and/ or the second hinge device are formed as film hinges. In particular, the first and second film hinges are formed in one piece of material together with the segments, i.e. the segments of the bending section and the film hinges, preferably all of them, are formed in one piece of material. In particular, the first hinge device and/ or the second hinge device are formed as webs of material bridging gaps or slits between the respectively adjacent segments. This allows a particularly space-saving construction and particularly cost-efficient manufacturing and assembly of the endoscope.

The cable lumen portion is preferably offset from the endoscope axis and, further preferably, a central axis of the cable lumen portion extends parallel to the endoscope axis. In particular, the cable lumen portion is arranged in a radially outer area of the bending section. Preferably, only a thin wall portion of the segments separates the cable lumen portion from a radially outer side of the segments, i.e. no other elements are received in the segments radially outside of the cable lumen portion. Thus, the endoscope will allow maximizing of a radial distance of the electrical cable with respect to the endoscope axis, thus leaving more room for a working channel accommodated in the bending section. This makes it possible to advantageously provide a large working channel even with small endoscopes and allows insertion of relatively large instruments through said working channel. In particular, the endoscope may comprise a working channel extending through the segments of the bending section.

Preferably, a wall thickness of the segments in or near the cable plane, particularly in the circumferential area of the cable lumen portion, further particularly adjacent to the cable lumen portion on a radially outer side thereof, is thinner than a thickness of a hinge element of the at least one first hinge device and/ or the at least one second hinge device. Expressed in other words, the cable lumen portion may be arranged between the first and second hinge devices directly in the circumferential direction. I.e. the cable lumen portion may overlap with the first and second hinge devices when viewed along the circumferential direction. This is possible, since very little stress is applied to the electrical cable and the segments in the area of the neutral plane or cable plane. Thus, material cost can advantageously be reduced and an available space for a working channel may be increased further.

Preferably, each of the at least one first hinge device and/ or the at least one second hinge device comprises two hinge elements, with one of said hinge elements being arranged on one side of the first bending plane and the other one of the hinge elements being arranged on the other side of the first bending plane, preferably such that the two hinge elements are arranged mirror symmetrically with respect to the first bending plane. Expressed in other words, each of the one or more first hinge devices may have two hinge elements connecting adjacent ones of the segments and/ or each of the one or more second hinge devices may have two hinge elements connecting adjacent ones of the segments. Each hinge element may e.g. be formed as a film hinge or by a web of material extending between the adjacent segments. The first hinge axis may extend between the hinge elements of the first hinge device. The second hinge axis may extend between the hinge elements of the second hinge device. Preferably, the hinge elements are arranged such that the first hinge axis and/ or the second hinge axis extend parallel to the cable plane or the neutral plane. Thus, the bending section may have a particularly simple structure, which is simple and cost-efficient in manufacture.

Expressed in other words, between each adjacent ones of the segments, two radially opposite cuts or gaps or slits are provided, which separate the segments from each other, with the first or second hinge device, in particular the respective hinge elements, separating the radially opposite cuts from each other. In particular, circumferential ends of each two radially opposite cuts or gaps or slits face each other in the circumferential direction. In particular, the radially opposite cuts or gaps or slits form circumferential ends, i.e. do not extend further than the circumferential ends, which face each other in the circumferential direction. In other words, the cuts or gaps or slits will not overlap in the axial direction and do not have portions extending circumferentially next to each other. Preferably, one of the radially opposite cuts or gaps or slits extends further through the bending section than the endoscope axis in order to provide an offset of the first or second hinge devices or the respective hinge elements from the cable plane or neutral plane.

Preferably, the segments form a major lumen portion, which has a larger cross-sectional area than the cable lumen portion and is further preferably laterally connected to the first channel structure. In other words, the cable lumen portion and the major lumen portion together may provide an essentially pear-shaped lumen. That is, no additional wall portion may separate the major lumen portion and the cable lumen portion. Thus, more space for accommodation for a working channel is available and a larger working channel may be used. However, an embodiment where the major lumen portion and the cable lumen portion are separated by a wall portion could also be provided. That is, a solution where the cable lumen is closed off from the major lumen portion to avoid slipping of the cable on a working channel surface can be provided.

Further preferably, in a transitional lumen wall area connecting the cable lumen portion and the major lumen portion, a lumen wall is formed convexly. In other words, the transitional lumen wall area bulges or protrudes radially inwards with respect to the endoscope axis. I.e. the transitional lumen wall extends further inwards than a line being tangential with respect to the cable lumen portion and the major lumen portion. The transitional lumen wall area may be a wall portion formed by the segment in an area where the cable lumen portion and the cable lumen portion are merged. Preferably, the transitional wall area provides a lateral support for the electrical cable. Due to this, the electrical cable may be supported on a radially inwards facing side by the working channel and adjacent to said working channel, particularly on all other sides, by a wall formed by the segments. Advantageously, a risk that the electrical cable may slip into the major lumen portion between a wall of said major lumen portion and the working channel accommodated therein may be minimized and a support for the electrical cable may be optimized.

Preferably, the major lumen portion is arranged in a cross-sectional area comprising the endoscope axis. In particular, the major lumen extends from the cable lumen portion to a diametrically opposite radially outer wall of the segments. Thus, a size of the major lumen may be optimized and a larger working channel may be used. Further preferably, the major lumen portion extends along an axis extending in the cable plane, preferably offset from the endoscope axis. Therefore, a size of the major lumen portion may be further increased.

Preferably, the segments further form at least one steering wire lumen portion for accommodation of at least one steering wire, said steering wire lumen portion being arranged at one side of the cable plane, i.e. offset from the cable plane. Due to this, efficient force transmission via the steering wires for bending the bending section and swivelling the distal tip unit in the first bending plane is possible. Preferably, the at least one steering wire lumen portion is arranged in or near the first bending plane, i.e. offset from the first bending plane at a maximum angle of 45°, further preferably 30°, further preferably 10°. Two such steering wire lumen portions may be arranged on opposing sides of the cable plane, preferably mirror symmetrically, with respect to the cable plane. Moreover, the at least one steering wire lumen portion may preferably be laterally connected to the major lumen portion and/ or to the cable lumen portion. In particular, the at least one steering wire lumen portion may be directly connected to the major lumen portion, i.e. not via the cable lumen portion or other lumen portions. Advantageously, this allows further increasing of the major lumen portion. Alternatively the at least one steering wire lumen may be closed off from the major lumen and/ or the cable lumen portion, i.e. may be separated via a wall portion.

Preferably, the endoscope is a two-way bending endoscope. I.e. the bending section of the endoscope has only one bending plane, which is the first bending plane. In this case, the cable plane is not near or coinciding with another bending plane and compressing or lengthening of the electrical cable during operation of the bending section will be effectively minimized. Thus, this two-way bending endoscope allows realizing the advantages of the present application particularly well, particularly with regard to reducing a risk of damaging the electrical cable during bending of the bending section.

Alternatively preferred, the endoscope is a four-way bending endoscope. That is, the endoscope has exactly two bending planes, the first bending plane and a second bending plane, which are preferably orthogonal to each other. In this case, the cable plane preferably corresponds to the second bending plane. In particular, the second bending plane may be defined by third hinge devices connecting a number of the segments. The third hinge devices may be arranged in the first bending plane for a particularly simple design. Alternatively, the second bending plane may be defined by the third hinge devices and by fourth hinge devices connecting respectively adjacent segments, the third and fourth hinge devices being alternatingly offset with respect to the first bending plane in a manner similar to that described above with regard to the first and second hinge devices and their offset from the cable plane.

Preferably, the first and second hinge devices and the third, and optionally fourth, hinge devices may be arranged alternatingly. In other words, along the axial direction (i.e. along the endoscope axis), a first one of the segments may be connected to a subsequent second one of the segments via one hinge device for bending in the first bending plane (i.e. one of the first and second hinge devices) and the second one of the segments may be connected to a subsequent third one of the segments via one hinge device for bending in the second bending plane (i.e. one of the third and optionally fourth hinge devices), and the third one of the segments may be connected to a subsequent fourth one of the segments via another hinge device for bending in the first bending plane (i.e. the other one of the first and second hinge devices), and the fourth one of the segments may be connected to a subsequent fifth one of the segments via another hinge device for bending in the second bending plane (i.e. another third hinge device or the other one of the third and fourth hinge devices). For example, along the axial direction, a first hinge device may be followed by a third hinge device, which may be followed by a second hinge device, which may be followed by another third or a fourth hinge device, and then again a first hinge device, and (optionally) so on.

For the sake of better clarity, optionally, the first hinge device may be called a first plane-one hinge device (i.e. a first hinge device for bending the bending section in the first bending plane) and the second hinge device may be called a second plane-one hinge device (i.e. another hinge device for bending the bending section in the first bending plane). Further, for the sake of clarity, the third hinge device may be called a first plane-two hinge device (i.e. a first hinge device for bending the bending section in the second bending plane). Yet further, in case there are third and fourth hinge devices as discussed above, the fourth hinge device may be called a second plane-two hinge device (i.e. another hinge device for bending the bending section in the second bending plane).

Preferably, the electrical cable is fixed at the distal tip unit and/ or at a distal end portion of the bending section. Further preferably, the electrical cable is loosely held in the bending section, preferably in the entire bending section. In other words, the electrical cable is preferably not fixed to the cable lumen portion of the segments of the bending section. This advantageously allows axial shifting of the electrical cable at least within the bending section. In this manner, the shifting of the electrical cable may balance out minimal lengthening and compressing, which may be applied to the electrical cable between each two segments during bending of the bending section due to the offset of the first and second hinge devices from the cable plane.

Further preferably, at a distal end segment of the segments, i.e. at a most distal one of the segments, a steering cable is held or anchored. In particular, the most distal one of the segments may form a wire holder or wire attachment portion for holding or attaching the at least one steering wire. Said wire holder may simply provide a distally facing stop surface for a stopper element connected to a distal end of the respective steering wire. This provides a simple design of the bending section, facilitates assembly of the endoscope and allows a direct and efficient appliance of operational force for bending the bending section. The most distal one of the segments may further form a mounting portion for connection of the distal tip unit. Further, the most proximal one of the segments may form a mounting portion for connection of the insertion tube.

Further, the object underlying the present application is solved by a system comprising an above-described endoscope and a monitor connectable to the endoscope.

### Brief description of figures

The following figures illustrate exemplary embodiments of the disclosure. The disclosure is not limited to the embodiments described below. Other embodiments, combinations of embodiments and modifications may be provided within the scope of protection defined by the claims.
Fig. 1 shows a schematic view of a system comprising an endoscope and a monitor according to the present disclosure.
Fig. 2 shows a perspective view of a proximal end of a bending section of a first embodiment of the present disclosure.
Fig. 3 shows a cross-sectional view through the bending section along a first hinge device of the first embodiment.
Fig. 4 shows a cross-sectional view through the bending section along a second hinge device of the first embodiment.
Fig. 5 shows frontal view of a most distal segment of the bending section of the first embodiment.
Fig. 6 shows a side view of the bending section orthogonal to a first bending plane B of the first embodiment.
Fig. 7 shows a side view of the bending section orthogonal to a cable plane C of the first embodiment.
Fig. 8 shows a simplified perspective view of a bending section of a second embodiment.
Fig. 9 shows a cross-sectional view of the bending section in an area of a third hinge device of the second embodiment.

### Detailed description of preferred embodiments

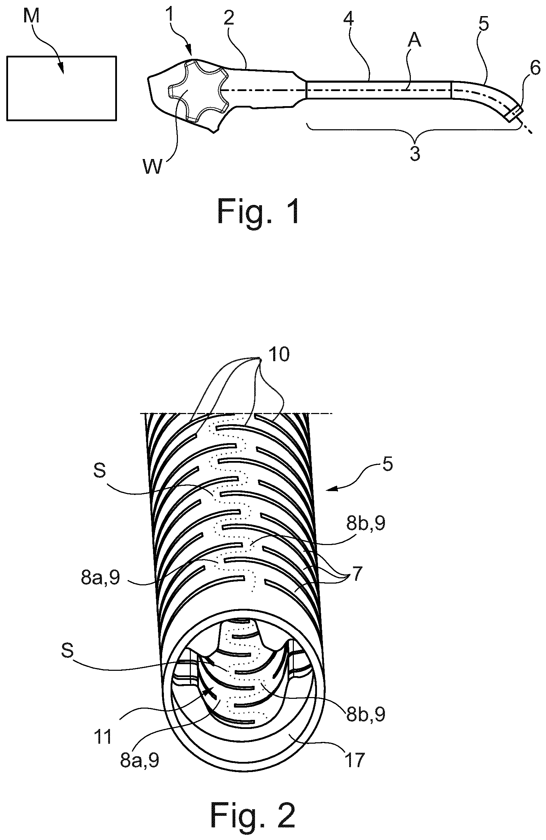

Fig. 1 shows a schematic view of an endoscope 1, which is preferably a single-use endoscope. The endoscope 1 has a proximal endoscope handle 2 and an insertion cord 3 extending distally from the endoscope handle 2 along an endoscope axis A. The insertion cord 3 has an insertion tube 4 connected to the endoscope handle 2. The insertion cord 3 further includes a bending section 5 connected to a distal end of the insertion tube 4 and a distal tip unit 6 connected to a distal end of the bending section 5.

At the distal tip unit 6, image capturing means such as a miniature video camera and illuminating means such as light-emitting diodes or optical fibres connected to a proximal source of light are arranged/installed, such that the patient's body cavity can be illuminated and inspected. An image captured by the image capturing means can be shown on a monitor M. The monitor M is provided separately from and connectable with the endoscope 1.

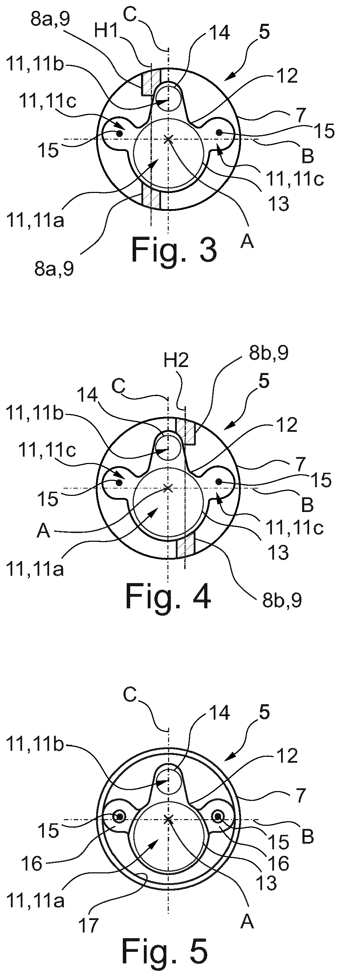

The bending section 5 is configured to perform a bending/ pivoting/ swivelling movement in at least two different directions/ in at least a first bending plane B shown in Fig. 3, Fig. 4 and Fig. 5 and described in more detail below. This enables a steering of the endoscope 1. The bending/ pivoting/ swivelling movement is controlled by a steering mechanism described in more detail below. In a neutral state, the bending section 5 is unbent by the steering mechanism, preferably straight/ in alignment with the insertion tube 4. When the bending section 5 is bent in the first bending plane B, a length of the bending section 5 on an inner curvature side is shortened. Further, a length of the bending section 5 on an outer curvature side is lengthened. Intermediately, there is a plane in which a length of the bending section 5 remains the same in the neutral state and in a bent state of the bending section 5. Said plane is called neutral or central plane C and is illustrated in Fig. 3, Fig. 4 and Fig. 5. The neutral or central plane C is orthogonal to the first bending plane B and intersects said first bending plane B along the endoscope axis A.

Fig. 2 shows a perspective view of a proximal portion of the bending section 5 of an embodiment of the present disclosure. The bending section 5 comprises a tubular wall, which forms a number of essentially ring-shaped segments 7. Said segments 7 are aligned along the endoscope axis A. The segments 7 are connected via hinge devices 8a, 8b, which are explained in more detail below with further reference to Fig. 3 and Fig. 4. The tubular wall including the segments 7 and the hinge devices 8a, 8b is formed in one piece of material. Each of the hinge devices 8a, 8b comprises two hinge elements 9. Other than at the hinge devices 8a, 8b, the segments 7 are separated by gaps or slits 10 extending essentially perpendicular to the endoscope axis A. Further, the segments 7 form a lumen 11 extending along the endoscope axis A and being described in more detail below with reference to Fig. 3 and Fig. 4. Each segment 7 forms part of said lumen 11.

The hinge devices 8a, 8b include a number of first hinge devices 8a and second hinge devices 8b. Each hinge element 9 is formed by a connecting web extending between and connecting adjacent ones of the segments 7. Fig. 3 shows a cross-sectional view through the bending section 5 along one of the first hinge devices 8a and Fig. 4 shows a cross-sectional view through the bending section 5 along one of the second hinge devices 8b.

The segments 7 shown respectively in Fig. 3 and Fig. 4 are formed by the tubular wall forming a lumen 11. The lumen 11 forms a continuous hollow with several lumen portions. Said lumen portions include a major lumen portion 11a, which is the largest among the lumen portions. The major lumen portion 11a e.g. forms or is configured to accommodate a working channel 13 for insertion of surgical instruments, flushing or the like. The working channel 13 is schematically shown in Fig. 3, Fig. 4 and Fig. 5. A central axis of the major lumen portion 11a extends in the cable plane C. A cross-sectional area of the major lumen portion 11a comprises the endoscope axis A. Further, the central axis of the major lumen portion 11a is offset from the endoscope axis A, such that the major lumen portion is arranged further to one side of the bending section 5.

Further, the lumen portions include a cable lumen portion 11b, which accommodates or is configured to accommodate an electrical cable 14, e.g. for control and/ or energy supply of an optical unit/ camera module at the distal tip unit 6. The electrical cable 14 is schematically shown in Fig. 3, Fig. 4 and Fig. 5. The cable lumen portion 11b is preferably connected to the major lumen portion 11a as shown in this embodiment, and has a central axis extending in the cable plane C. Further, the central axis of the cable lumen portion 11b is offset from the endoscope axis A to a side opposite of the central axis of the major lumen portion 11a. A cross-sectional area or diameter of the cable lumen portion 11b is smaller than that of the major lumen portion 11a. In transitional lumen wall areas 12 connecting the cable lumen portion 11b and the major lumen portion 11a on each side, a lumen wall is formed convexly, i.e. bulges towards the endoscope axis A. Thus, a cross-section of the major lumen portion 11a and the cable lumen portion 11b together is essentially pear-shaped.

Additionally, the lumen portions include at least two steering wire lumen portions 11c, which accommodate or are configured to accommodate corresponding steering wires 15 schematically shown in Fig. 3, Fig. 4 and Fig. 5. The steering wires 15 are part of the steering mechanism for controlling the bending of the bending section 5. A proximal end of the steering wires 15 is connected to a control wheel W arranged in or at the endoscope handle 2 for operating the steering wires 15. The steering wire lumen portions 11c are preferably connected to the major lumen portion 11a and the cable lumen portion 11b on opposing sides with respect to the cable plane C, as shown in the present embodiment. In particular, one of the steering wire lumen portions 11c is arranged at or near one of the transitional areas 12 and the other one of the steering wire lumen portions 11c is arranged at or near the other one of the transitional areas 12. Further in particular, central axis of the steering wire lumen portions 11c are arranged in or near the first bending plane B. A cross-sectional area or diameter of each steering wire lumen portion 11c is smaller than that of the major lumen portion 11a.

Fig. 3 further shows the first hinge device 8a. Said first hinge device 8a is formed by two hinge elements 9, which are webs of material extending between adjacent ones of the segments 7 to connect these segments 7. In particular, the hinge elements 9 form film hinges. The hinge elements 9 of the first hinge device 8a are arranged on opposite sides with respect to the first bending plane B, particularly at mirror symmetrical locations with respect to the first bending plane B. Further, the hinge elements 9 of the first hinge device 8a are offset from the neutral or centre plan C to one side, i.e. both to the same side, of the cable plane C. The first hinge device 8a, particularly its hinge elements 9, define a first hinge axis H1. In the present embodiment, said first hinge axis H1 is parallel to the cable plane C.

Fig. 4 further shows the second hinge device 8b. Said second hinge device 8b is formed by two hinge elements 9, which are webs of material extending between adjacent ones of the segments 7 to connect these segments 7. In particular, the hinge elements 9 form film hinges. The hinge elements 9 of the second hinge device 8b are arranged on opposite sides with respect to the first bending plane B, particularly at mirror symmetrical locations with respect to the first bending plane B. Further, the hinge elements 9 of the second hinge device 8b are offset from the neutral or centre plan C to another side of the cable plane C, i.e. to a side opposite of the side to which the hinge elements 9 of the first hinge device 8a are offset. Both hinge elements 9 of the second hinge device 8b are offset to the same side of the cable plane C. The second hinge device 8b, particularly its hinge elements 9, define a second hinge axis H2. In the present embodiment, said second hinge axis H2 is parallel to the cable plane C and to the first hinge axis H1.

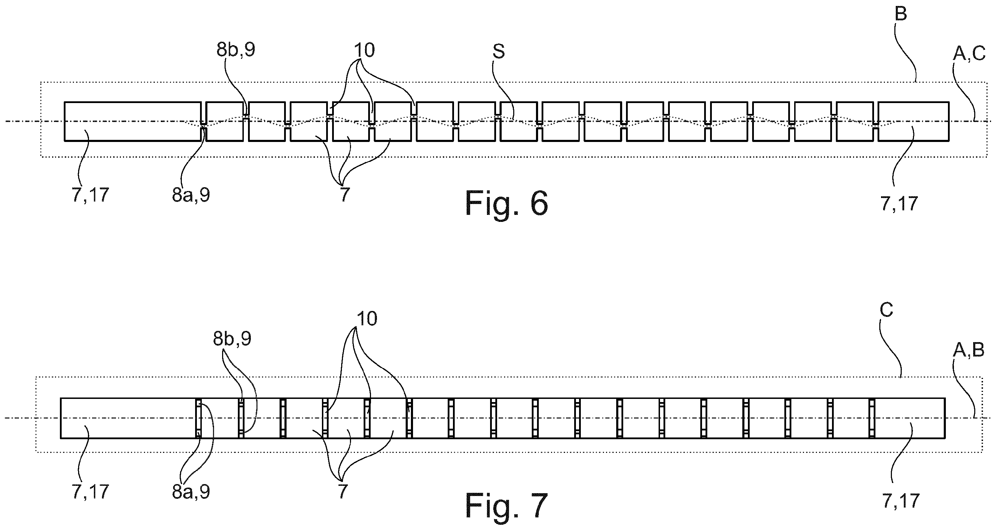

Due to the above described offset of the first hinge device 8a and the second hinge device 8b to opposite sides of the cable plane C, the bending section essentially has a spine S extending along the endoscope axis, i.e. in a proximal-distal direction, while meandering back and forth through/ over the cable plane C. The segments 7 extend in a rib-like manner from said meandering spine S. Further, due to the hinge devices 8a, 8b each having two hinge elements 9 on opposite sides of the first bending plane B, two such meandering spines S are provided. These spines S are indicated by a dotted line in Fig. 2 and Fig. 6, with Fig. 6 showing a side view orthogonal to the first bending plane B. Further, due to the above described mirror-symmetrical arrangement of the two hinge elements 9 of each hinge device 8a, 8b with respect to the first bending plane B, two such meandering spines S are formed by the bending section 5 that meander essentially parallel to each other, when viewed in a side view orthogonal to the first bending plane B. In the side view orthogonal to the first bending plane B shown in Fig. 6, the hinge elements 9 of the one spine S are respectively hidden behind the hinge elements 9 of the other spine 9.

Fig. 5 shows a frontal view of a most distal segment of the segments 7 of the bending section 5. Essentially, shapes in a cross-sectional view of said most distal segment of the segments 7 and the lumen 11 formed therein are the same as those of the other segments 7, except for the following: Each steering wire lumen portion 11c comprises a wire holder 16 where the respective steering wires 15 are attached in order to apply a steering force to the bending section 5. In the present embodiment, the wire holders 16 are formed as flanges or bulges or collars, e.g. clamping the steering wires 15 or providing a stop for a distal end element or portion of the steering wires 15. Further, the most distal segment of the segments 7 forms a mounting portion 17 for mounting the distal tip unit 6. The most proximal segment of the segments 7 also forms a mounting portion 17 for mounting the bending section 5 to the insertion tube 4. In the present embodiment, both of said mounting portions 17 are formed as a sleeve-like receptacle. Such mounting portions 17 are illustrated in Fig. 2, where the most proximal segment is shown in a perspective view or in Fig. 5 where the most distal segment is shown in a frontal view. Due to said mounting portion 17, the most distal one and/ or the most proximal one of the segments 7 may be longer in a direction of the endoscope axis A than the other segments 7, as can be seen in Fig. 6 and Fig. 7. Fig. 7 shows a side view orthogonal to the neutral or central plane C.

The segments 7 of the bending section 5 are alternatingly connected by first hinge devices 8a and second hinge devices 8b. I.e. a first one of the segments is connected to a distally adjacent second one of the segments 7 via a first hinge device 8a, and said second one of the segments 7 is connected to another distally adjacent third one of the segments 7 via a second hinge device 8b. Said third one of the segments 7 is in turn connected to yet another distally adjacent fourth one of the segments 7 via a first hinge device 8a, etc.

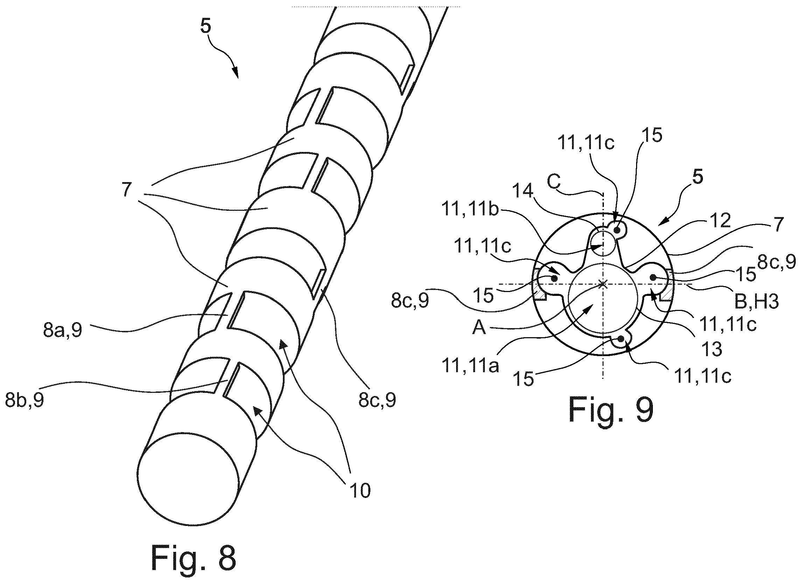

Fig. 8 and Fig. 9 show a second preferred embodiment of the present disclosure, wherein the endoscope is a four-way bending endoscope. Therein, the bending section 5 is adapted to bend in two bending planes, the first bending plane B and a second bending plane. The second bending plane preferably corresponds to the neutral or central plane C. Said neutral or central plane C serves as a neutral or central plane, where an axial length of the bending section 5 is the same in the neutral state or when bent, only during bending of the bending section 5 in the first bending plane.

The endoscope 1, particularly the segments 7 including the first hinge device 8a and the second hinge device 8b as well as the lumen 11 and the cables etc. accommodated therein, essentially corresponds to the first embodiment, except for differences explained below, and the previous figures and description are referred to. In addition to the first and second hinge devices 8a, 8b, a bending section 5 of the second embodiment has third hinge devices 8c, which connect some of the segments 7 to each other. In particular, the segments 7 are alternatingly connected via first hinge devices 8a, second hinge devices 8b and third hinge devices 8c as shown in Fig. 8. In particular, Fig. 8 shows a simplified perspective view of a bending section 5 according to the second embodiment, wherein the lumen 11 is not shown.

Other arrangements of the hinge devices may be provided. For example, along the axial direction, between each first and second hinge devices 8a, 8b, one third hinge device 8c (or optionally fourth hinge device 8d) may be arranged. That is, a first one of the segments 7 may be connected to a subsequent second one of the segments 7 via the first hinge device 8a (also called a first plane-one hinge device). The second one of the segments 7 may be connected to a subsequent third one of the segments 7 via the third hinge device 8c (also called a first plane-two hinge device) for bending in the second bending plane. The third one of the segments 7 may be connected to a subsequent fourth one of the segments 7 via the second hinge device 8b (also called a second plane-one hinge device) for bending in the first bending plane. The fourth one of the segments 7 may be connected to a subsequent fifth one of the segments 7 via the another third hinge device 8c or via the fourth hinge device 8d (also called a second plane-two hinge device) for bending in the second bending plane. This order may be repeated.

Fig. 9 shows a cross-sectional view of the bending section in an area of one of the third hinge devices 8c. The third hinge devices 8c have two hinge elements 9 structured similar to those of the first and second hinge devices 8a, 8b, but arranged diametrically opposite to each other in the second bending plane. That is, the third hinge device 8c defines a third hinge axis H3, which extends in the first bending plane, orthogonally to the first and second hinge devices. Further, the lumen 11 forms two additional steering wire lumens 11c, which are arranged on opposing sides of the first bending plane B, particularly in or near the second bending plane, and accommodate additional steering wires 15 for controlling a bending of the bending section 5 in the second bending plane.

### List of reference numbers

- 1: Endoscope
- 2: Endoscope handle
- 3: Insertion cord
- 4: Insertion tube
- 5: Bending section
- 6: Distal tip unit
- 7: Segments
- 8a, 8b: First hinge devices and second hinge devices
- 9: Hinge elements
- 10: Gaps or slits
- 11: lumen
- 11a: major lumen portion
- 11b: cable lumen portion
- 11c: steering wire lumen portions
- 12: transitional areas
- 13: working channel
- 14: electrical cable
- 15: steering wires
- 16: wire holder
- 17: mounting portion

- A: Endoscope axis
- B: Bending plane
- C: Neutral or central plane / second bending plane
- H1: First bending axis
- H2: Second bending axis
- H3: Third bending axis

## Claims

1. An endoscope (1) comprising:
a proximal endoscope handle or interface (2);
an insertion cord (3) extending along an endoscope axis (A) from the endoscope handle or interface (2) and configured to be inserted into a patient's body cavity, the insertion cord (3) comprising an insertion tube (4), a bending section (5) and a distal tip unit (6); and
a steering mechanism (8) configured to swivel the distal tip unit (6) by bending the bending section (5) at least in a first bending plane (B); wherein
the bending section (5) comprises a number of segments (7) aligned along the endoscope axis (A) and forming a cable lumen portion (11b) for accommodation of at least one electrical cable (14), said cable lumen portion (11b) being offset from the endoscope axis (A) and arranged in a cable plane (C), wherein the cable plane (C) is a neutral or central plane being orthogonal to the first bending plane and intersecting said first bending plane along the endoscope axis, and
the segments (7) are connected by at least one first hinge device (8a) and at least one second hinge device (8b), which define the first bending plane (B) and are configured to allow bending of the bending section in the first bending plane (B), with at least one of the segments (7) being connected to a respectively adjacent one of the segments (7) by a first hinge device (8a) defining a first hinge axis (H1), which is offset from the cable plane (C) in a first direction **,** when viewed in a cross-section through the bending section along the first hinge device,
and at least another one of the segments (7) being connected to a respectively adjacent one of the segments (7) by a second hinge device (8b) defining a second hinge axis (H2), which is offset from the cable plane (C) in a second direction opposite to the first direction, when viewed in a cross-section through the bending section along the second hinge device.

2. The endoscope (1) according to claim 1, wherein the first hinge device (8a) is offset from the cable plane (C) by less than half a diameter of the bending section (5), preferably maximum 40 %, further preferably by maximum 30 %, further preferably by maximum 20% of the diameter of the bending section (5).

3. The endoscope (1) according to one of the preceding claims, wherein along the endoscope axis (A), adjacent ones of the segments (7) are alternatingly connected by first hinge devices (8a) and second hinge devices (8b).

4. The endoscope (1) according to one of the preceding claims, wherein the first hinge device (8a) and/ or the second hinge device (8b) form film hinges and are formed in one piece of material together with the segments (7).

5. The endoscope (1) according to one of the preceding claims, wherein a wall thickness of the segments (7) in the cable plane (C), particularly adjacent to the cable lumen portion (11b), is thinner than a thickness of a hinge element (9) of the at least one first hinge device (8a) and/ or the at least one second hinge device (8b).

6. The endoscope (1) according to claim 5, wherein each of the at least one first hinge device (8a) and/ or the at least one second hinge device (8b) respectively comprises two hinge elements (9), with one of said two hinge-elements (9) being arranged on one side of the first bending plane (B) and the other one of said two hinge elements (9) being arranged on the other side of the first bending plane (B), preferably such that the two hinge elements (9) are arranged mirror symmetrically with respect to the first bending plane (B).

7. The endoscope (1) according to one of the preceding claims, wherein the segments (7) form a major lumen portion (11a), which has a larger cross-sectional area than the cable lumen portion (11b) and is laterally connected to the cable lumen portion (11b).

8. The endoscope (1) according to claim 7, wherein in a transitional lumen wall area (12) connecting the cable lumen portion (11b) and the major lumen portion (11a), a lumen wall is formed convex.

9. The endoscope (1) according to one of the preceding claims 7 and 8, wherein the major lumen portion (11a) is arranged in a cross-sectional area comprising the endoscope axis (A).

10. The endoscope (1) according to one of the preceding claims, wherein the segments (7) further form at least one steering wire lumen portion (11c) for accommodation of at least one steering wire (15), said steering wire lumen portion (11c) being arranged at one side of the cable plane (C).

11. The endoscope (1) according to one of the preceding claims, wherein the endoscope (1) is a two-way bending endoscope, the distal tip unit (6) being steerable only in the first bending plane (B).

12. The endoscope (1) according to one of the preceding claims1 to 10, wherein the endoscope (1) is a four-way bending endoscope, the distal tip unit (6) being steerable in the first bending plane and in a second bending plane defined at least by third hinge devices connecting a number of the segments (7), and preferably the cable plane (C) serves as the second bending plane.

13. A system comprising an endoscope (1) according to any one of the claims 1 to 12 and a monitor (M) connectable to the endoscope (1).

## Patentansprüche

1. Endoskop (1) mit
einem proximalen Endoskopgriff oder einer Schnittstelle (2);
einem sich von dem Endoskopgriff oder der Schnittstelle (2) entlang einer Endoskopachse (A) erstreckenden und zum Einführen in eine Körperhöhle eines Patienten ausgebildeten Einführstrang (3), wobei der Einführstrang (3) ein Einführrohr (4), einen Biegeabschnitt (5) und eine distale Spitzeneinheit (6) aufweist; und
einem Steuermechanismus (8), der dazu ausgebildet ist, die distale Spitzeneinheit (6) durch Biegen des Biegeabschnitts (5) zumindest in einer ersten Biegeebene (B) zu verschwenken; wobei
der Biegeabschnitt (5) eine Anzahl von entlang der Endoskopachse (A) ausgerichteten Segmenten (7) aufweist, die einen Kabellumenabschnitt (11b) zur Aufnahme wenigstens eines elektrischen Kabels (14) bilden, wobei der Kabellumenabschnitt (11b) gegenüber der Endoskopachse (A) versetzt und in einer Kabelebene (C) angeordnet ist, wobei die Kabelebene (C) eine zu der ersten Biegeebene orthogonale Neutral- oder Mittelebene ist, welche die erste Biegeebene entlang der Endoskopachse schneidet, und
die Segmente (7) durch wenigstens eine erste Gelenkeinrichtung (8a) und wenigstens eine zweite Gelenkeinrichtung (8b) verbunden sind, welche die erste Biegeebene (B) definieren und dazu ausgebildet sind, ein Biegen des Biegeabschnitts in der ersten Biegeebene (B) zu ermöglichen, wobei wenigstens eines der Segmente (7) mit einem jeweils benachbarten Segment (7) durch eine erste Gelenkeinrichtung (8a) verbunden ist, die eine erste Gelenkachse (H1) definiert, welche, in einem Querschnitt durch den Biegeabschnitt entlang der ersten Gelenkeinrichtung betrachtet, gegenüber der Kabelebene (C) in einer ersten Richtung versetzt ist,
und wobei wenigstens ein weiteres der Segmente (7) mit einem jeweils benachbarten Segment (7) durch eine zweite Gelenkeinrichtung (8b) verbunden ist, die eine zweite Gelenkachse (H2) definiert, welche, in einem Querschnitt durch den Biegeabschnitt entlang der zweiten Gelenkeinrichtung betrachtet, gegenüber der Kabelebene (C) in einer der ersten Richtung entgegengesetzten zweiten Richtung versetzt ist.

2. Endoskop (1) nach Anspruch 1, wobei die erste Gelenkeinrichtung (8a) gegenüber der Kabelebene (C) um weniger als die Hälfte eines Durchmessers des Biegeabschnitts (5), bevorzugt um höchstens 40 %, weiter bevorzugt um höchstens 30 %, noch weiter bevorzugt um höchstens 20 % des Durchmessers des Biegeabschnitts (5), versetzt ist.

3. Endoskop (1) nach einem der vorhergehenden Ansprüche, wobei entlang der Endoskopachse (A) benachbarte Segmente (7) abwechselnd durch erste Gelenkeinrichtungen (8a) und zweite Gelenkeinrichtungen (8b) verbunden sind.

4. Endoskop (1) nach einem der vorhergehenden Ansprüche, wobei die erste Gelenkeinrichtung (8a) und/oder die zweite Gelenkeinrichtung (8b) Filmscharniere bilden und zusammen mit den Segmenten (7) einstückig ausgebildet sind.

5. Endoskop (1) nach einem der vorhergehenden Ansprüche, wobei eine Wandstärke der Segmente (7) in der Kabelebene (C), insbesondere angrenzend an den Kabellumenabschnitt (11b), geringer ist als eine Dicke eines Gelenkelements (9) der wenigstens einen ersten Gelenkeinrichtung (8a) und/oder der wenigstens einen zweiten Gelenkeinrichtung (8b).

6. Endoskop (1) nach Anspruch 5, wobei jede der wenigstens einen ersten Gelenkeinrichtung (8a) und/oder der wenigstens einen zweiten Gelenkeinrichtung (8b) jeweils zwei Gelenkelemente (9) aufweist, wobei eines der zwei Gelenkelemente (9) auf einer Seite der ersten Biegeebene (B) und das andere der zwei Gelenkelemente (9) auf der anderen Seite der ersten Biegeebene (B) angeordnet ist, vorzugsweise derart, dass die zwei Gelenkelemente (9) bezüglich der ersten Biegeebene (B) spiegelsymmetrisch angeordnet sind.

7. Endoskop (1) nach einem der vorhergehenden Ansprüche, wobei die Segmente (7) einen Hauptlumenabschnitt (11a) bilden, der eine größere Querschnittsfläche als der Kabellumenabschnitt (11b) aufweist und seitlich mit dem Kabellumenabschnitt (11b) verbunden ist.

8. Endoskop (1) nach Anspruch 7, wobei in einem den Kabellumenabschnitt (11b) und den Hauptlumenabschnitt (11a) verbindenden Übergangslumenwandbereich (12) eine Lumenwand konvex ausgebildet ist.

9. Endoskop (1) nach einem der vorhergehenden Ansprüche 7 und 8, wobei der Hauptlumenabschnitt (11a) in einem die Endoskopachse (A) aufweisenden Querschnittsbereich angeordnet ist.

10. Endoskop (1) nach einem der vorhergehenden Ansprüche, wobei die Segmente (7) ferner wenigstens einen Steuerdrahtlumenabschnitt (11c) zur Aufnahme wenigstens eines Steuerdrahts (15) bilden, wobei der Steuerdrahtlumenabschnitt (11c) auf einer Seite der Kabelebene (C) angeordnet ist.

11. Endoskop (1) nach einem der vorhergehenden Ansprüche, wobei das Endoskop (1) ein in zwei Richtungen biegsames Endoskop ist, wobei die distale Spitzeneinheit (6) ausschließlich in der ersten Biegeebene (B) steuerbar ist.

12. Endoskop (1) nach einem der vorhergehenden Ansprüche 1 bis 10, wobei das Endoskop (1) ein in vier Richtungen biegsames Endoskop ist, wobei die distale Spitzeneinheit (6) in der ersten Biegeebene und in einer zweiten Biegeebene steuerbar ist, die zumindest durch dritte Gelenkeinrichtungen definiert ist, welche eine Anzahl der Segmente (7) miteinander verbinden, und wobei die Kabelebene (C) vorzugsweise als die zweite Biegeebene dient.

13. System mit einem Endoskop (1) nach einem der Ansprüche 1 bis 12 und einem mit dem Endoskop (1) verbindbaren Monitor (M).

## Revendications

1. Endoscope (1) comprenant :
une poignée ou une interface d'endoscope proximale (2) ;
un cordon d'insertion (3) s'étendant le long de l'axe de l'endoscope (A) à partir de la poignée ou de l'interface de l'endoscope (2) et configuré pour être inséré dans une cavité corporelle du patient, le cordon d'insertion (3) comprenant un tube d'insertion (4), une section de flexion (5) et une unité d'extrémité distale (6) ; et
un mécanisme de direction (8) configuré pour faire pivoter l'unité d'extrémité distale (6) en pliant la section de flexion (5) au moins dans un premier plan de flexion (B) ; dans lequel
la section de flexion (5) comprend un certain nombre de segments (7) alignés le long de l'axe de l'endoscope (A) et formant une portion de lumière de câble (11b) destinée à accueillir au moins un câble électrique (14), ladite portion de lumière de câble (11b) étant décalée par rapport à l'axe de l'endoscope (A) et agencée dans un plan de câble (C), dans lequel le plan de câble (C) est un plan neutre ou central orthogonal au premier plan de flexion et coupant ledit premier plan de flexion le long de l'axe de l'endoscope, et
les segments (7) sont reliés par au moins un premier dispositif de charnière (8a) et au moins un deuxième dispositif de charnière (8b), qui définissent le premier plan de flexion (B) et sont configurés pour permettre la flexion de la section de flexion dans le premier plan de flexion (B), avec au moins un des segments (7) relié à un segment (7) respectivement adjacent par un premier dispositif de charnière (8a) définissant un premier axe de charnière (H1), qui est décalé par rapport au plan du câble (C) dans une première direction, lorsqu'il est observé en coupe transversale à travers la section de flexion le long du premier dispositif de charnière,
et au moins un autre des segments (7) étant connecté à un segment (7) respectivement adjacent par un deuxième dispositif de charnière (8b) définissant un deuxième axe de charnière (H2), qui est décalé par rapport au plan du câble (C) dans une deuxième direction opposée à la première direction, lorsqu'il est vu en coupe transversale à travers la section de flexion le long du deuxième dispositif de charnière.

2. Endoscope (1) selon la revendication 1, dans lequel le premier dispositif de charnière (8a) est décalé du plan du câble (C) de moins de la moitié du diamètre de la section de flexion (5), de préférence de 40 % au maximum, et de préférence de 30 % au maximum, plus préférentiellement d'au maximum 20 % du diamètre de la section de flexion (5).

3. Endoscope (1) selon l'une des revendications précédentes, dans lequel, le long de l'axe de l'endoscope (A), les segments adjacents (7) sont reliés alternativement par des premiers dispositifs de charnière (8a) et des deuxièmes dispositifs de charnière (8b).

4. Endoscope (1) selon l'une des revendications précédentes, dans lequel le premier dispositif de charnière (8a) et/ou le deuxième dispositif de charnière (8b) forment des charnières de film et sont formés d'une seule pièce de matériau avec les segments (7).

5. Endoscope (1) selon l'une des revendications précédentes, dans lequel l'épaisseur de la paroi des segments (7) dans le plan du câble (C), en particulier à proximité de la portion de lumière du câble (11b), est plus mince que l'épaisseur d'un élément de charnière (9) du au moins un premier dispositif de charnière (8a) et/ou du au moins un deuxième dispositif de charnière (8b).

6. Endoscope (1) selon la revendication 5, dans lequel chacun des au moins un premier dispositif de charnière (8a) et/ou des au moins un deuxième dispositif de charnière (8b) comprend respectivement deux éléments de charnière (9), l'un des deux éléments de charnière (9) étant agencé d'un côté du premier plan de flexion (B) et l'autre des deux éléments de charnière (9) étant agencé de l'autre côté du premier plan de flexion (B), de préférence de telle sorte que les deux éléments de charnière (9) soient disposés symétriquement par rapport au premier plan de flexion (B).

7. Endoscope (1) selon l'une des revendications précédentes, dans lequel les segments (7) forment une portion de lumière principale (11a), qui présente une surface en coupe transversale plus grande que la portion de lumière du câble (11b) et est connectée latéralement à la portion de lumière du câble (11b).

8. Endoscope (1) selon la revendication 7, dans lequel dans une zone de paroi de lumière de transition (12) reliant la portion de lumière du câble (11b) et la portion de lumière principale (11a), une paroi de lumière est formée de manière convexe.

9. Endoscope (1) selon l'une des revendications précédentes 7 et 8, dans lequel la portion principale de la lumière (11a) est agencée dans une zone en coupe transversale comprenant l'axe de l'endoscope (A).

10. Endoscope (1) selon l'une des revendications précédentes, dans lequel les segments (7) forment en outre au moins une portion de lumière de fil de direction (11c) pour loger au moins un fil de direction (15), ladite portion de lumière de fil de direction (11c) étant agencée d'un côté du plan du câble (C).

11. Endoscope (1) selon l'une des revendications précédentes, dans lequel l'endoscope (1) est un endoscope à flexion bidirectionnelle, l'unité d'extrémité distale (6) étant orientable uniquement dans le premier plan de flexion (B).

12. Endoscope (1) selon l'une des revendications précédentes 1 à 10, dans lequel l'endoscope (1) est un endoscope à quatre voies de flexion, l'unité d'extrémité distale (6) étant orientable dans le premier plan de flexion et dans un deuxième plan de flexion défini au moins par des troisièmes dispositifs de charnière reliant un certain nombre de segments (7), et de préférence le plan du câble (C) sert de deuxième plan de flexion.

13. Système comprenant un endoscope (1) selon l'une quelconque des revendications 1 à 12 ; et un moniteur (M) pouvant être connecté à l'endoscope (1).
